# EUROPÄISCHE PATENTANMELDUNG

(11) **EP 3 293 125 A1**
(43) Veröffentlichungstag der Anmeldung: **14.03.2018**
(21) Anmeldenummer: 17189729.1
(22) Anmeldetag: 07.09.2017
(51) Int. Cl.: B65B 55/10, B65B 55/08, B65B 55/16, B65B 55/18, A61B 50/30, B65B 3/00

(54) **VERFAHREN UND VORRICHTUNG ZUR STERILEN HANDHABUNG EINES MEDIZINPRODUKTES**

(30) Priorität: 09.09.2016 DE 102016117025
(71) Anmelder: Plur, Andreas, 63571 Gelnhausen (DE)
(72) Erfinder: Plur, Andreas, 63571 Gelnhausen (DE)
(74) Vertreter: Stoffregen, Hans-Herbert

(57) **Zusammenfassung**

Die Erfindung betrifft ein Verfahren und eine Vorrichtung zur sterilen Handhabung eines Medizinproduktes, insbesondere einer Zytostatika-Zubereitung. Das Verfahren umfasst die Verfahrensschritte:
-Einbringen eines Medizinproduktes in eine erste, vorzugsweise sterilisierbare und/oder vorsterilisierte Schutzverpackung in einer Reinraumumgebung,
-Verschließen der ersten Schutzverpackung in der Reinraumumgebung,
-vorzugsweise Einbringen der ersten Schutzverpackung mit dem Medizinprodukt in eine zweite Schutzverpackung und Verschließen der zweiten Schutzverpackung,
-Einbringen der ersten und ggf. zweiten Schutzverpackung in eine Umverpackung und Verschließen der Umverpackung zu einem Verpackungs-Set.

Um eine einfache und sichere Handhabung des Medizinproduktes zu ermöglichen, ist vorgesehen, dass das aus der zumindest ersten und vorzugsweise zweiten Schutzverpackung sowie der Umverpackung gebildete Verpackungs-Sets in eine mittels Wischdesinfektion desinfizierbare Endverpackung eingeschweißt wird.

## Beschreibung

Die Erfindung bezieht sich auf ein Verfahren zum sterilen Handhabung eines Medizinproduktes, insbesondere einer Zytostatika-Zubereitung nach dem Oberbegriff des Anspruchs 1 sowie auf eine Vorrichtung zur sterilen Handhabung eines Medizinproduktes nach dem Oberbegriff des Anspruchs 5.

Eine Vorrichtung zur sicheren und sterilen Handhabung wie Verpackung, Transport, Lagerung, Transfer zum Patienten sowie interner steriler Transport von sterilen Medizinprodukten, insbesondere Zytostatika-Zubereitungen wird von der KWP-Kunststoff-Werk-Plur GmbH unter der Bezeichnung Zyto-Sterilset® (eingetragene Marke) angeboten. Das Sterilset umfasst eine erste Schutzverpackung zur Aufnahme der sterilen Zytostatika-Zubereitung in Form eines Flachbeutels mit Druckverschluss bzw. eines Siegelrandbeutels mit Selbstklebeverschluss. Die erste Schutzverpackung mit der sterilen Zytostatika-Zubereitung wird in eine zweite Schutzverpackung eingebracht, die schließlich in einer dritten Schutzverpackung als Umverpackung eingebracht wird.

Die erste Schutzverpackung kann aus einer Folie aus z. B. Low-Density-Polyethylen (LDPE) oder aus einer Kombination eines medizinischen Papiers bzw. eines Kunststoffinaterials wie TYVEK® (eingetragene Marke) und Folie hergestellt sein.

Die aus erster Schutzverpackung, zweiter Schutzverpackung sowie Umverpackung bestehenden Verpackungs-Sets können nur dann mittels Gamma-Sterilisation bzw. Ethylenoxid (ETO)-Sterilisation sterilisiert werden, sofern Schutzverpackungen verwendet werden, die zumindest abschnittsweise aus TYVEK®-Folie hergestellt sind.

Derartige Zytostatika-Schutzverpackungen werden insbesondere in Reinraum- und Steril-Apotheken bei der Herstellung sowie zentralen Versorgung von Zytostatika verwendet.

Nach dem Stand der Technik besteht die Umverpackung aus einer Kombination aus einer Folie mit medizinischem Papier, d. h. einem mit einem Kunststoff beschichteten Papier oder aus einer Kombination aus einer Folie mit einem Kunststoffmaterial wie TYVEK (eingetragene Marke). Folglich wird eine Bag-in-Bag (BIB)-Reinraumverpackung bereitgestellt.

Eine derartige Außenverpackung kann vor dem Einschleusen in eine Reinraumumgebung nicht mittels Wischdesinfektion desinfiziert werden, da erwähntermaßen die Endverpackung zumindest abschnittsweise ein medizinisches Papier bzw. ein Kunststoffmaterial wie TYVEK aufweist, welches eine Wischdesinfektion nicht zulässt.

Die DE 10 2007 047 623 A1 betrifft ein Verfahren und eine Vorrichtung zum sterilen Verpacken von Medizinprodukten. Dabei wird ein erstes Medizinprodukt in eine gasdichte Schutzverpackung eingebracht, die anschließend verschlossen wird. Die gasdichte Schutzverpackung wird sodann mit dem ersten Medizinprodukt und mindestens einem zweiten Medizinprodukt in einer Verpackung eingebracht, die anschließend verschlossen wir. Sodann wird die Schutzverpackung mit dem zweiten Medizinprodukt in der Endverpackung mit Hilfe eines Gases sterilisiert. Die Schutzverpackung ist zumindest abschnittsweise aus einer mit Metall beschichteten Kunststofffolie hergestellt. Die Endverpackung ist zumindest abschnittsweise aus medizinischen Papier oder einem Kunststoffmaterial wie TYVEK (eingetragene Marke) hergestellt.

Auch bei dieser Verpackung ist eine Wischdesinfektion vor Einschleusen der Verpackung in eine Reinraumumgebung nicht möglich.

Davon ausgehend liegt der vorliegenden Erfindung die Aufgabe zugrunde, ein Verfahren und eine Vorrichtung der eingangs genannten Art derart weiterzubilden, dass eine einfache und sichere Handhabung ermöglicht wird.

Die Aufgabe wird verfahrensmäßig u.a. dadurch gelöst, dass das aus der zumindest ersten und vorzugsweise zweiten Schutzverpackung sowie der Umverpackung gebildete Verpackungs-Sets in eine mittels Wischdesinfektion desinfizierbare Endverpackung eingeschweißt wird.

Durch die Einbringung des Verpackungs-Sets in eine mit Wischdesinfektion desinfizierbare Endverpackung wird gegenüber dem Stand der Technik der Vorteil erreicht, dass die Endverpackung mit Wischdesinfektion gereinigt und anschließend (Bag-in-Bag) in eine Reinraumumgebung eingeschleust werden kann. Dadurch ist sichergestellt, dass durch die Endverpackung keine Verunreinigungen in die Reinraumumgebung gelangen.

Durch die Verwendung eines strahlensterilisationsfähigen, Ethylenoxid durchlässigen und niedertemperatursterilisationsfähigen Materials für die erste, zweite und gegebenenfalls dritte Schutzverpackung als Umverpackung ist sichergestellt, dass das Verpackungs-Set mittels Gammastrahlung, Niedertemperatursterilisation und/oder mit Hilfe von Ethylenoxid (folgend kurz ETO) sterilisiert werden kann.

Die Aufgabe wird vorrichtungsmäßig u. a. dadurch gelöst, dass das Verpackungs-Set in einer aus einem mittels Wischdesinfektion desinfizierbaren Material hergestellten Endverpackung eingeschweißt ist.

Vorzugsweise ist die zumindest eine Schutzverpackung sowie die Umverpackung zumindest abschnittsweise aus einem strahlensterilisationsfähigen, Ethylenoxid durchlässigen und niedertemperatursterilisationsfähigen Kunststoffmaterial hergestellt.

Vorzugsweise ist die Endverpackung aus einer PE-Folie insbesondere Low-Density-Polyethylen-Folie hergestellt.

Die zumindest eine Schutzverpackung ist zumindest Abschnittsweise aus TYVEK (eingetragene Marke) hergestellt.

Durch die erfindungsgemäße Ausführungsform besteht die Möglichkeit, mehrere Produkte wie Flachbeutel mit Druckverschluss oder Siegelrandbeutel mit Selbstklebeverschluss in einem Verpackungs-Set mittels Gammastrahlung zu sterilisieren.

Auch besteht die Möglichkeit, Verpackungs-Sets in eine doppelte TYVEK- Verpackung zu verschließen.

Ferner besteht die Möglichkeit mehrere sterilisierbare oder vorsterilisierte erste Schutzverpackungen wie Flachbeutel offen ineinander zu schieben mit der Möglichkeit, ein derartiges Verpackens-Set mittels Gammastrahlung zu Sterilisieren.

Insgesamt ergibt sich eine erleichterte Anwendung bei kleineren Schutzverpackungen, so dass sowohl die Herstellung als auch die Anwendung wirtschaftlicher ist.

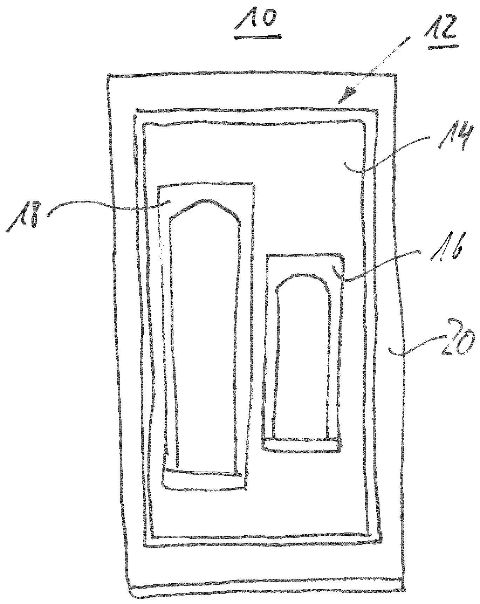

Weitere Einzelheiten, Vorteile und Merkmale der Erfindung ergeben sich nicht nur aus den Ansprüchen, den diesen zu entnehmenden Merkmalen - für sich und/oder in Kombination - , sondern auch aus der nachfolgenden Beschreibung eines der Zeichnung zu entnehmenden bevorzugten Ausführungsbeispiels.

Die einzige Figur zeigt eine Vorrichtung 10 zur sicheren Handhabung eines sterilen Medizinproduktes, insbesondere einer sterilen Zytostatika-Zubereitung 12. Im dargestellten Ausführungsbeispiel ist die sterile Zytostatika-Zubereitung in einer ersten Schutzverpackung 14 in Form eines Siegelrandbeutels mit Selbstklebeverschluss 16 eingebracht und in diesem verschlossen. Die erste Schutzverpackung ist wiederum in einer zweiten Schutzverpackung 18 in Form eines Siegelrandbeutels mit Selbstklebeverschluss 20 eingebracht und verschlossen. Sodann ist die verschlossene zweite Schutzverpackung 18 in einer dritten Schutzverpackung 22 als Umverpackung eingebracht und verschlossen.

Die erste, zweite und dritte Schutzverpackung 14, 18, 22 bilden ein mittels Strahlung sterilisierbares Verpackungs-Set 26, welches gemäß der Erfindung in einer desinfizierbaren, vorzugsweise mittels Wischdesinfektion desinfizierbaren Endeverpackung eingeschweißt wird. In der dargestellten Ausführungsform ist die Endverpackung 28 als PE-Folienbeutel hergestellt in geöffnetem Zustand dargestellt.

Die Schutzverpackungen 14, 18, 22 sind zumindest abschnittsweise aus einem strahlensterilisationsfähigen, Etylenoxid durchlässigen und niedertemperatursterilisationsfähigen Kunststoffmaterial hergestellt. Alternativ zu Siegelrandbeutel mit Selbstklebeverschluss kann die erste Schutzverpackung 14 auch als Flachbeutel mit Druckverschluss hergestellt sein. Als Materialien werden Low-Density-Polyethylen-Folien mit einem Kunststoffmaterial wie vorzugsweise TYVEK (eingetragene Marke) verwendet. Dadurch besteht die Möglichkeit, die Schutzverpackungen bzw. das Verpackungs-Set 26 vor Einbringen in die Endverpackung 28 zu sterilisieren.

Die Endverpackung ist aus einer PE-Folie vorzugsweise Low-Density-Polyethylen (LDPE) hergestellt. Nach Einbringen des Verpackungs-Sets 26 in die Endverpackung 28 wird diese randseitig verschweißt.

Bei der weiteren Handhabung der Vorrichtung 10 wird die Endverpackung 28 vor Einbringen der Endverpackung 28 in eine Reinraumumgebung durch Wischdesinfektion desinfiziert. Dadurch ist sichergestellt, dass Keime nicht in die Reinraumumgebung gelangen. In der Reinraumumgebung wird sodann die Endverpackung 28 geöffnet und das Verpackungs-Set aus der Endverpackung entnommen. Sodann wird aus der dritten Umverpackung 22 die zweite Umverpackung 18 und die erste Umverpackung 14 entnommen. Die erste Umverpackung 14 mit eingeschlossenem Medizinprodukt 12 wird für den Transfer zum Patienten bzw. den intern sterilen Transport aus der Reinraumumgebung ausgeschleust.

## Patentansprüche

1. Verfahren zur sterilen Handhabung eines Medizinproduktes, insbesondere einer Zytostatika-Zubereitung, umfassend die Verfahrensschritte:
- Einbringen eines Medizinproduktes in eine erste, vorzugsweise sterilisierbare und/oder vorsterilisierte Schutzverpackung in einer Reinraumumgebung,
- Verschließen der ersten Schutzverpackung in der Reinraumumgebung,
- vorzugsweise Einbringen der ersten Schutzverpackung mit dem Medizinprodukt in eine zweite Schutzverpackung und Verschließen der zweiten Schutzverpackung,
- Einbringen der ersten und ggf. zweiten Schutzverpackung in eine Umverpackung und Verschließen der Umverpackung zu einem Verpackungs-Set,
**dadurch gekennzeichnet,**
**dass** das aus der zumindest ersten und vorzugsweise zweiten Schutzverpackung sowie der Umverpackung gebildete Verpackungs-Sets in eine mittels Wischdesinfektion desinfizierbare Endverpackung eingeschweißt wird.

2. Verfahren nach Anspruch 1,
**dadurch gekennzeichnet,**
**dass** das Verpackungs-Set vor Einbringen in die Endverpackung mit Hilfe von Gammastrahlung sterilisiert wird

3. Verfahren nach Anspruch 1 oder 2,
**dadurch gekennzeichnet,**
**dass** das Verpackungs-Set vor Einbringen in die Endverpackung mit Hilfe von Ethylenoxid sterilisiert wird.

4. Verfahren nach zumindest einem der vorhergehenden Ansprüche,
**dadurch gekennzeichnet,**
**dass** das Verpackungs-Set mittels Niedertemperatursterilisation sterilisiert wird.

5. Vorrichtung (10) zur sterilen Handhabung eines Medizinproduktes (12), insbesondere einer Zytostatika-Zubereitung, umfassend zumindest ein Vepackungs-Set (26) mit zumindest einer Schutzverpackung (14, 18, 22) zur Aufnahme des Medizinproduktes (12) und einer die Schutzverpackung (14, 18, 22) aufnehmenden Umverpackung (28),
**dadurch gekennzeichnet,**
**dass** das Verpackungs-Set (26) in einer aus einem mittels Wischdesinfektion desinfizierbaren Material hergestellten Endverpackung (28) eingeschweißt ist.

6. Vorrichtung nach Anspruch 5,
**dadurch gekennzeichnet,**
**dass** die Endverpackung (28) aus einer PE-Folie hergestellt ist.

7. Vorrichtung nach Anspruch 5 oder 6,
**dadurch gekennzeichnet,**
**dass** die Endverpackung (28) verschweißt ist.

8. Vorrichtung nach zumindest einem der Ansprüche 5 bis 7,
**dadurch gekennzeichnet,**
**dass** die zumindest eine Schutzverpackung (14, 18, 22) aus einem mittels Strahlung wie Gamma-Strahlung, mittels Gas wie Ethylenoxid und/oder mittels Niedertemperaturstrahlung sterilisierbaren Material hergestellt ist.

9. Vorrichtung nach zumindest einem der Ansprüche 5 bis 9,
**dadurch gekennzeichnet,**
**dass** die zumindest eine Schutzverpackung (14, 18, 22) zumindest abschnittsweise aus TYVEK (eingetragene Marke) hergestellt ist.
